# EUROPEAN PATENT SPECIFICATION

(11) **EP 0 478 602 B1**
(45) Date of publication and mention of the grant of the patent: **03.01.1996**
(21) Application number: 90909052.4
(22) Date of filing: 20.06.1990
(51) Int. Cl.: C12N 15/31

(54) **EXPRESSION OF TETANUS TOXIN FRAGMENT C**
EXPRESSION VON TETANUSTOXINFRAGMENT C
EXPRESSION DU FRAGMENT C DE TOXINE TETANIQUE

(30) Priority: 20.06.1989 GB 8914122
(43) Date of publication of application: 08.04.1992
(73) Proprietor: EVANS MEDICAL LIMITED, Leatherhead, Surrey KT22 7PQ (GB)
(72) Inventor: FAIRWEATHER, Neil, Fraser, Kent BR3 3BS (GB); MAKOFF, Andrew, Joseph, Kent BR3 3BS (GB)
(74) Representative: Woods, Geoffrey Corlett
(86) International application number: PCT/GB90/00943
(87) International publication number: WO 90/15871

(56) References cited:
- EP-A- 0 209 281
- FR-A- 2 249 679
- THE EMBO JOURNAL, vol. 5, no. 10, IRL Press Ltd., Oxford (GB); U. EISEL et al., pp. 2495-2502
- ACTA PATH. MICROBIOL. IMMUNOL. SCAND., Sect. C.92, 1984; T.B. HELTING et al., pp. 59-63
- INFECTION & IMMUNITY, vol. 55, no. 11, November 1987, American Society for Microbiology; N.F. FAIRWEATHER et al., pp. 2541-2545
- BIOTECHNOLOGY, vol. 7, October 1989, Research Papers; A.J. MAKOFF et al., pp. 1043-1046
- INFECTION & IMMUNITY, vol. 58, no. 4, April 1990, American Society for Microbiology; J.L. HALPERN et al., pp. 1004-1009

## Description

The present invention relates to the production of tetanus toxin C fragment.

E.coli has been successfully used as a host in which many foreign proteins have been obtained in large amounts. However, it has been found that the foreign protein is generally produced in an insoluble form, despite being soluble in its native environment (J.Biochem., 1986, 240, 1-12). When viewed by a microscope under phase contrast conditions, the protein aggregates are visible within the bacterial cells and are usually referred to as inclusion or refractile bodies.

It is relatively straightforward on a laboratory scale to separate inclusion bodies from the majority of E.coli proteins. This can be achieved for example by centrifugation and can lead to remarkable purification in just one step. However, on an industrial scale purification of the inclusion bodies is difficult to perform. In any event, to obtain the protein in an active form, the inclusion bodies have to be solubilized, requiring the use of strong denaturing agents such as urea or guanidinium chloride. The denaturant is then removed under conditions which allow the protein to refold into its native conformation. These conditions are established empirically for each protein and frequently involve the use of very dilute solutions, which on scale-up can lead to enormous volumes and consequent process engineering problems. These difficulties can be offset to some extent by the high productivity of E.coli, but clearly it would be desirable to obtain this benefit with the production of the protein in soluble form.

Vaccination is generally effective in the prevention of tetanus infection in most Western countries, although incomplete vaccination in some third world countries can account for up to 1 million cases of tetanus every year. Current tetanus vaccines are produced by formaldehyde treatment of tetanus toxin obtained by culture of the causative bacterium C.tetani under anaerobic conditions. It has been suggested that impurities arising during the formaldehyde treatment are partly responsible for the adverse effects sometimes seen with tetanus toxoid. In any event, production of the toxoid is technically difficult since it necessitates the total removal of tetanus toxin by, for example, repeated cycles of affinity purification.

The structural gene for tetanus toxin has been cloned and sequenced (Fairweather et al, J. Bacteriol. 165, 1986, 21-27; Fairweather and Lyness, Nuc. Acid Res. 14, 1986, 7809-7812). These studies have confirmed the structure of tetanus toxin as a 150kD protein comprising 1315 amino acids. Fragment C is a 50kD polypeptide generated by papain cleavage of the toxin and comprises, or substantially corresponds to, the 451 amino acids at the C-terminus. Fragment C has also been expressed in E.coli (Fairweather et al, 1986; Fairweather et al, Infection and Immunity 55, 1987, 2541-2545; EP-A-0209281) fused to part of the E.coli trpE protein or to fragment B of tetanus toxin. In this regard, E.coli strain DH1 containing pTet12, which encodes fragment C fused to part of trpE and fragment B, was deposited at the National Collection of Type Cultures, London, UK on the 28th June 1985 under accession number NCTC 11918. These fusions were all insoluble in the cytoplasm of E.coli cells.

Fragment C has been shown to be non-toxic and is capable of immunising mice and guinea pigs (Helting and Zwisler, J. Biol. Chem. 252, 1977, 187-193; Helting and Nau, Act. Pathol. Microbiol. Scan. Sect. C 92, 1984, 59-63).

It has now been found that, when fragment C of tetanus toxin is expressed in E.coli not fused to any foreign or native polypeptide, the resulting product is substantially soluble and is not produced as inclusion bodies. Additionally, high levels of expression of the product can be obtained, and the product is obtained in a substantially active form, as judged by biochemical and immunological criteria.

Accordingly, the invention provides a process for producing fragment C of tetanus toxin in soluble form, which process comprises:
(i) culturing an Escherichia coli host cell transformed with an expression vector comprising a coding sequence consisting of a Met start codon followed by a sequence encoding fragment C, wherein said culturing is effected under conditions such that said coding sequence is expressed and fragment C thereby produced in soluble form; and
(ii) recovering said soluble fragment C from the cytoplasm of said Escherichia coli host cell.

The invention also provides an Escherichia coli expression vector comprising a coding sequence consisting of a Met start codon followed by a sequence encoding fragment C of tetanus toxin. The DNA encoding mature fragment C is incorporated in the vector in the correct reading frame under the control of appropriate transcriptional and translational control elements. Examples of such elements include a promoter and a transcriptional terminator site. An operator and/or attenuator sequence may also be present upstream of the coding sequence. Typically the expression vector is a plasmid. The promoter may be the lac, trp, λPL, λPR, lpp, T7ø10 or tac promoter. Use of an inducible promoter, such as the tac promoter, is preferred as it enables expression to be more readily controlled. Expression of the tac promoter is induced by isopropyl-β-D-thiogalactoside (IPTG).

The invention includes an Escherichia coli host cell transformed with an expression vector as described above. Examples of strains of E.coli for use with the present invention include HB101, DH1 and MM294. The strain of E.coli may be transformed with the expression vector and the transformed strain cultured using procedures known in the art.

Fragment C may be recovered from the cytoplasm (in which it is dissolved) of the E.coli cells by lysis of the cells followed by centrifugation, acid precipitation and ion exchange chromatography. Typically the cells from a growing culture, for example from a 2.5 litre culture, are harvested by centrifugation and resuspended in a suitable buffer (e.g. 25mM Tris/HCl pH8.0, 150mM) NaCl containing phenylmethylsulphonyl fluoride (PMSF)). Cells are lysed by sonication, by passage through a French Pressure Cell, or by treatment with a non-ionic detergent (e.g. Nonidet NP40) and lysozyme. The lysate is centrifuged and the resulting supernatant isolated. The supernatant, containing fragment C, may then be purified using ion exchange chromatography. Fractions containing fragment C are collected and further purified until the desired degree of purity is obtained. Such further purification may involve affinity purification using monoclonal or polyclonal antibodies. Generally the degree of purity is at least 80%, preferably at least 90% and more preferably at least 95%.

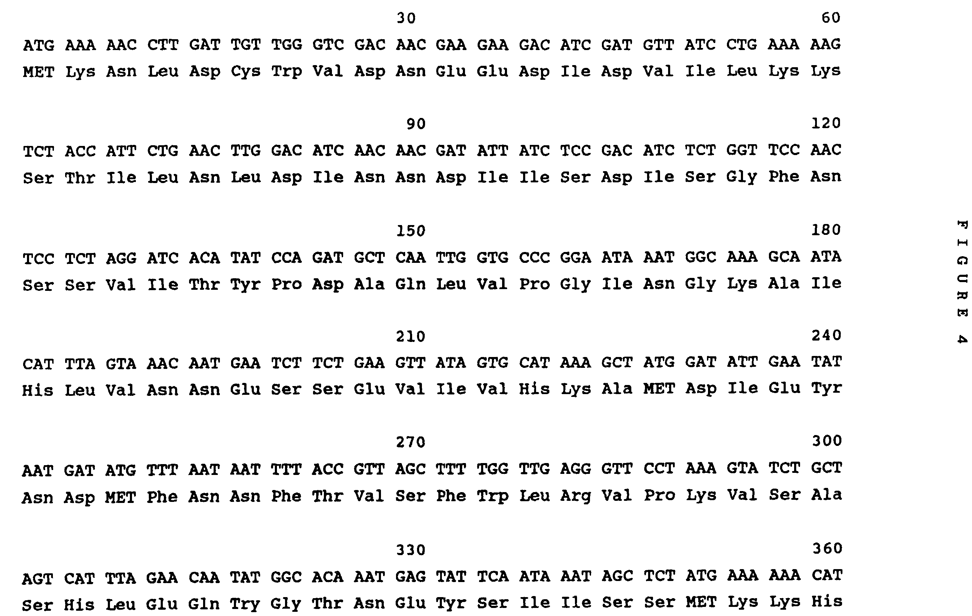

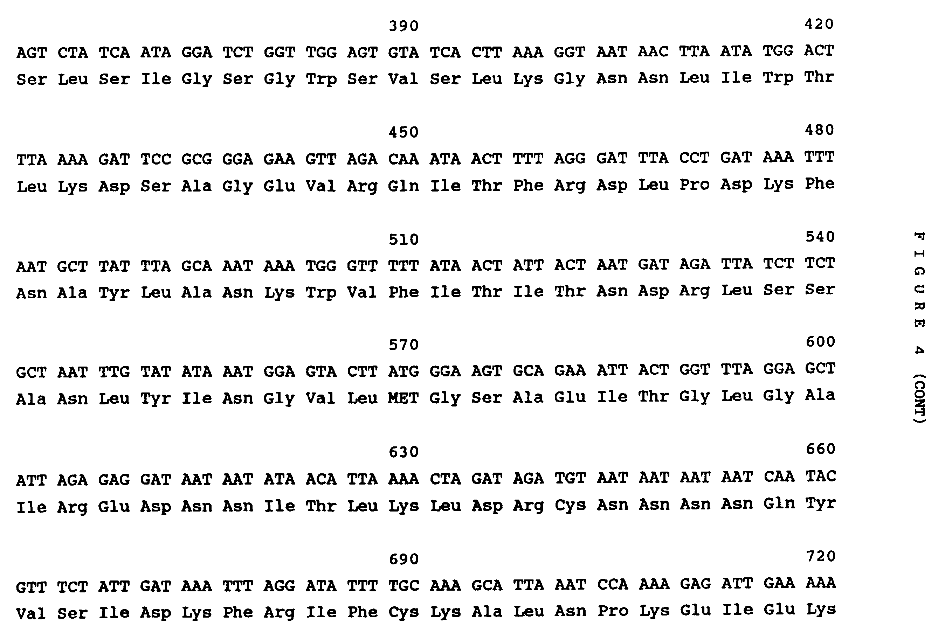

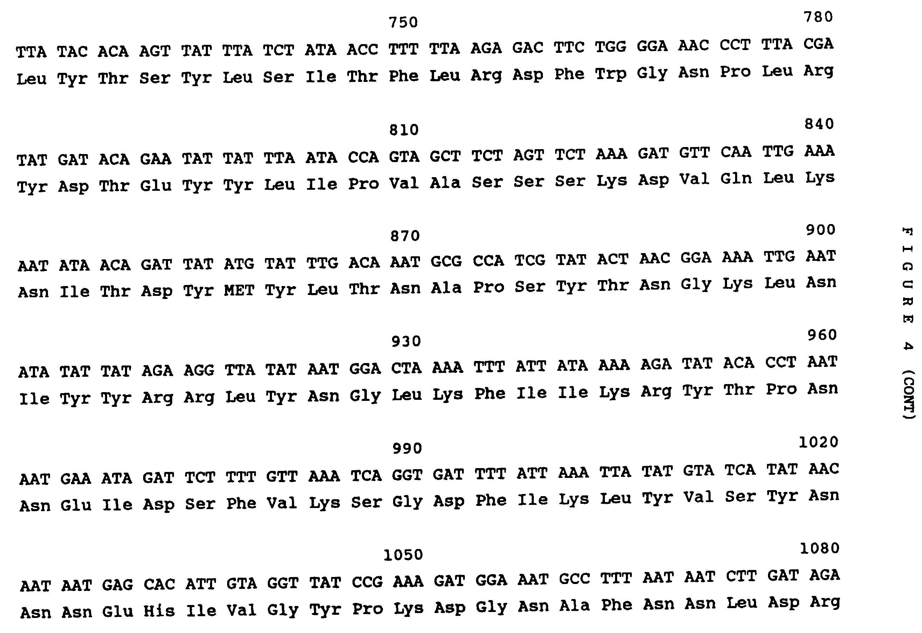

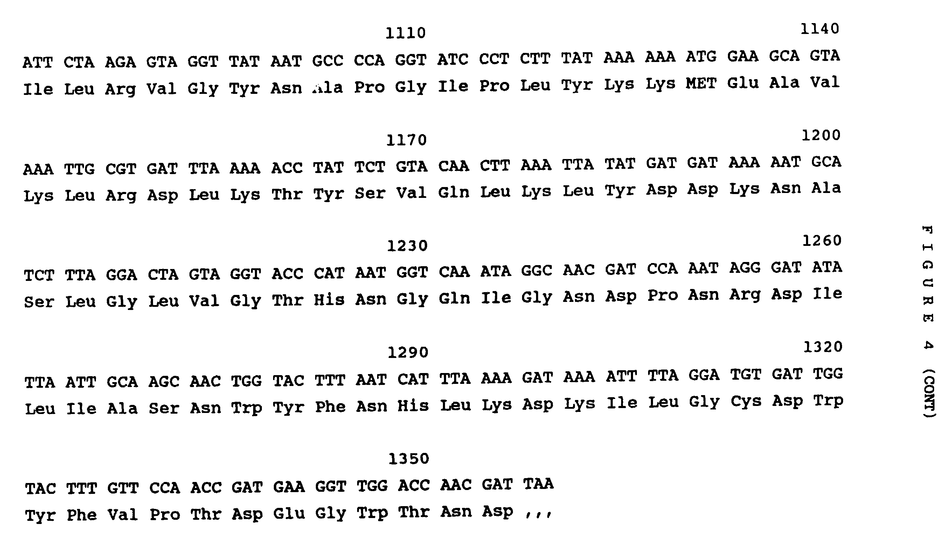

As indicated earlier, fragment C is a 50kD polypeptide derived from the C-terminus of tetanus toxin. The amino acid sequence of the fragment C produced according to the invention may comprise that known for fragment C or a substantially similar sequence having substantially the same biological and immunological properties. In particular, the amino acid sequence is that set forth in Figure 4 or has at least 90%, preferably 95%, more preferably 98%, homology with that set forth in Figure 4. Thus, within these limitations the sequence may be varied by one or more amino acid substitutions, extensions, insertions and/or deletions provided the resulting polypeptide retains substantially the same biological and immunological properties as fragment C. In the case of amino acid substitutions, one or more of the amino acid residues of fragment C may thus be replaced by one or more other amino acid residues. Candidate substitutions include Ser for Thr and vice versa, Glu for Asp and vice versa, Gln for Asn and vice versa, Leu for Ile and vice versa, Ala for Val and vice versa and Arg for Lys and vice versa.

Variations within the amino acid sequence of fragment C may of course be obtained by effecting changes in the DNA sequence encoding fragment C. This may be achieved by, for example, the use of restriction endonucleases, insertion of oligonucleotide linkers, exonucleases and/or polymerases and site-directed mutagenesis.

The fragment C produced according to the invention may be included in a vaccine for conferring immunity to tetanus. The vaccine typically comprises the fragment C and a pharmaceutically acceptable carrier or diluent. The vaccine may include other antigens to provide a multi-valent vaccine. Typically carriers and diluents are sterile, pyrogen-free liquid media suitable as vehicles for introducing a polypeptide into a patient. Isotonic saline solution may be employed.

The vaccine may also comprise an adjuvant for stimulating the immune response and thereby enhancing the effect of the vaccine. A convenient adjuvant is aluminium hydroxide. Conveniently the vaccines are formulated to contain a final concentration of fragment C or its derivative of from 0.2 to 200 µg/ml, preferably 5 to 50 µg/ml, most preferably about 30 µg/ml. After formulation the vaccine may be incorporated into a sterile container which is then sealed and stored at a low temperature, for example 4°C, or it may be freeze-dried.

The vaccines may be administered by any conventional method for the administration of vaccines including oral and parenteral (e.g. subcutaneous or intramuscular) injection. The treatment may consist of a single dose of vaccine or a plurality of doses over a period of time. It is recommended that each dose is 0.1 to 2 ml preferably 0.2 to 1 ml, most preferably about 0.5 ml of vaccine.

The following Example illustrates the present invention. A Comparative Example is provided. E.coli MM294 harbouring plasmid pTETtac1 was deposited at the National Collection of Industrial and Marine Bacteria, Aberdeen, GB on 13 June 1989 under accession number NCIMB 40154. In the accompanying drawings:

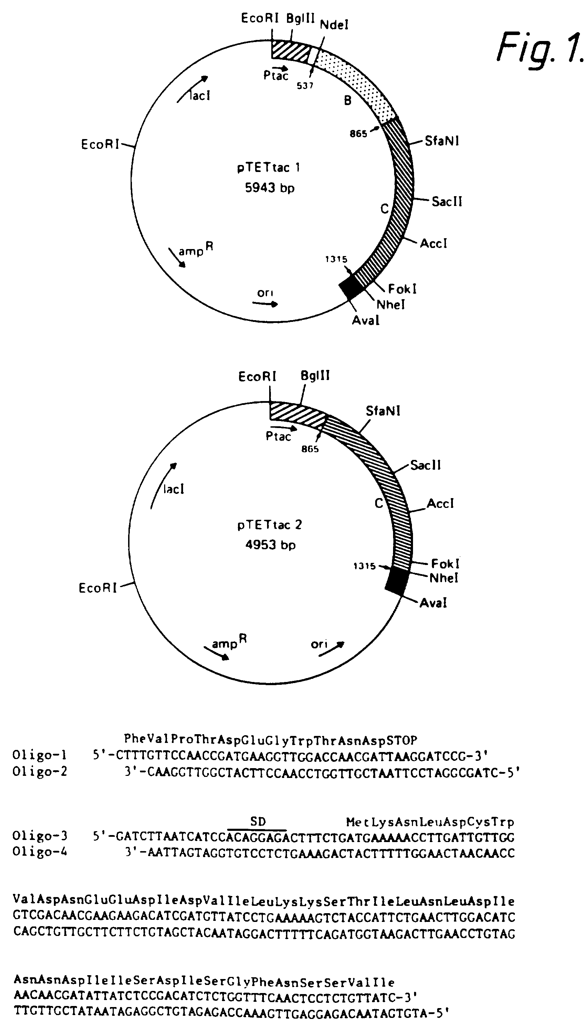

Figure 1 shows the construction of expression plasmids pTETtac1 (a) and pTETtac2 (b) and the oligonucleotides used (c). In (a) pTETtac1 is shown as consisting of the tac promoter and modified trpD ribosome binding site 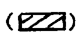 ; the first 3 codons of the coding sequence of γ-IF (▭); the coding regions of fragments B 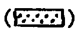 and C 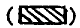 of tetanus toxin; and the trpA terminator 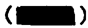 . The numbers arrowed refer to amino acid positions in tetanus toxin.

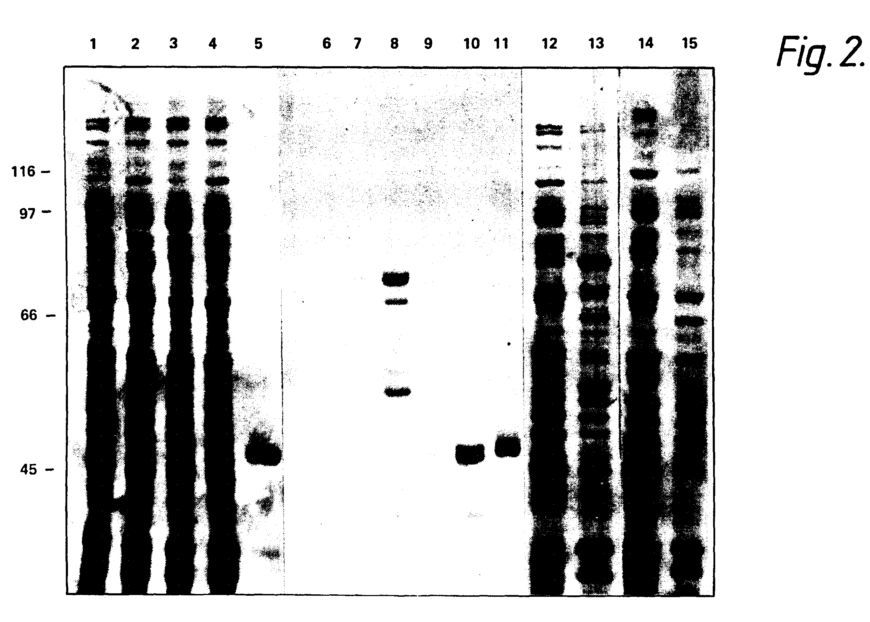

Figure 2 shows SDS-polyacrylamide gels analysis of E.coli extracts containing pTETtac1 and pTETtac2. Lanes 1-5 and 12-15 are Coomassie blue stained; 6-11 are Western blots. Total cell extracts from pTETtacl uninduced (lanes 1,7) and induced (lanes 2,8), pTETtac2 uninduced (lanes 3,9) and induced (lanes 4,10). Control extract (lane 6). Fragment C from C.tetani (lanes 5,11), pTETtac1 induced supernatant (lane 12) and pellet (lane 13). pTETtac2 induced supernatant (lane 14) and pellet (lane 15).

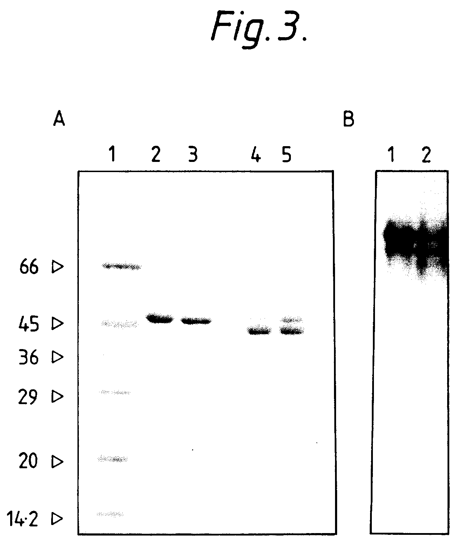

Figure 3 shows the analysis of recombinant and C.tetani derived fragment C by SDS-PAGE (Figure 3A) and by non-denaturing PAGE (Figure 3B). Samples for SDS-PAGE electrophoresis were prepared by addition to an equal volume of 2 x sample buffer (14% SDS, 20% glycerol, 10% 2-mercapto-ethanol and 0,125M Tris-HCl pH 6.8). Mercaptoethanol was omitted for non-reduced samples. Samples were heated at 100°C for 5 mins before loading. Figure 3A: Lane 1, protein size markers (in kDa); Lane 2,3 reduced samples; Lane 4, 5 non-reduced samples; Lane 2,4 recombinant fragment C; Lane 3,5 C.tetani derived fragment C. Each lane contains 5 µg fragment C. Gel was stained with Coomassie brilliant blue. Samples for non-denaturing PAGE were diluted to a final concentration of 50mM Na Cl, 10% glycerol, 5mM Tris/HCl pH 7.5. Gels were run as described by Ballantine and Boxer, J. Bacteriol. 163, 454-459, 1985. Figure 3B: Lane 1, recombinant fragment C; Lane 2, C.tetani-derived fragment C. Each lane contains 15 µg fragment C.

Figure 4 shows the DNA and amino acid sequence of fragment C of tetanus toxin.

### EXAMPLE

### 1. EXPERIMENTAL PROTOCOL

### Construction of plasmids

Expression plasmid pTETtac1 was constructed from plasmid pPFtac1, a derivative of pIFGtac124A (Makoff and Smallwood, Biochem. Soc. Trans. 16, 1988, 48-49). The NdeI - NheI coding region of pPFtac1 was replaced by tetanus toxin coding sequence (Figure 1). Most of this coding sequence was provided by two restriction fragments: a NdeI AccI 1858bp fragment from pTet1 and an AccI - FokI 442bp fragment from pTet8 (Fairweather et al, 1986). The rest of the sequence was encoded by a pair of synthetic oligonucleotides both 44 bases long, where the codon bias was optimized for expression in E.coli. These oligonucleotides are oligos 1 and 2 shown in Fig 1.

Plasmid pTETtac2 was constructed from pTETtac1 by replacing the Bg1II - SfaNI region of pTETtac1 by a pair of synthetic oligonucleotides each 161 nucleotides long (Fig 1). These oligonucleotides reproduced the sequence upstream of the initiation codon and optimised the coding sequence, at the beginning of the C fragment region, for expression in E.coli (Fig 1). The oligonucleotides were ligated to the 302 bp SfaNI - SacII and 4490bp SacII - Bg1II fragments of pTETtac1.

The oligonucleotides for both constructions were synthesized in a Pharmacia Gene Assembler and purified by electrophoresis on a denaturing polyacrylamide gel. Their 5' ends were phosphorylated by T4 polynucleotide kinase before ligation. Ligated products were transformed into E.coli strain MM294 (Meselson and Yuan, Nature 217, 1988, 1110-1114). The sequences specified by the oligonucleotides were confirmed by direct sequencing of both plasmids pTETtac1 and pTETtac2 by the chain termination method (Sanger et al, Proc. Nat1. Acad. Sci. USA 74, 1977, 5463-5467) using primers which were synthesized and purified in the same way as the oligonucleotides.

### Induction and Analysis of Expressed Proteins

Cultures of E.coli MM294 containing the above plasmids were induced as described previously (Makoff et al, J. Gen. Microbiol. 135, 1989, 11-24) with minor alterations. Cultures were grown overnight in L broth containing 100 µg/ml ampicillin, and diluted 1 in 5 into fresh L-broth also containing 100 µg/ml ampicillin. Isopropyl-β-D-thiogalactoside (IPTG) was added to 60 µg/ml to induce expression of fragment C. The culture was harvested after 4 hours and total cell extracts were analysed on 7.5% sodium dodecyl sulphate (SDS)-polyacrylamide gels as described previously (Makoff et al, 1989). Western blots were detected by rabbit antisera against fragment C isolated from C. tetani and goat antirabbit IgG conjugated to horse radish peroxidase followed by Colour Development Reagent (Biorad) and hydrogen peroxide.

In order to determine if the expressed proteins were soluble, the equivalent of 1ml cells at an OD650 of 1.0 were incubated at 0°C for 20 mins in 100 µl 50mM Tris-HCl pH8.0, 50mM NaCl, 5% glycerol, 1mM phenylmethylsulphonylfluoride (PMSF) containing 400 µg lysozyme followed by four successive freeze-thaw cycles. Viscosity was reduced by shearing the DNA by rapid passage through a 1ml syringe attached to a 0.8mm bore needle. The extracts were centrifuged for 10 min at 12 k x g at 4°C and the pellets resuspended in the same volume of the above buffer. Both pellet fractions and supernatants were centrifuged again and the resuspension repeated. Samples were diluted into 2 x Laemmli loading buffer and 20 µl of each fraction was loaded onto an SDS-polyacrylamide gel and analysed as above.

### Immunisation of mice

Vaccines containing fragment C from E.coli or C.tetani were prepared with 15% w/v Alhydrogel (Trade Mark). Dilutions were made as indicated in Table 1 and 0.5 ml was injected subcutaneously into NIH mice. Mice were challenged four weeks later with approximately 100 LD₅₀s tetanus toxin, and those mice surviving after four days were considered protected.

### Purification of Recombinant Fragment C

Cell pellets (9.6g wet wt) from 2.5 litres of culture were resuspended in about 35 mls of 25mM Tris/HC1 pH8., 150mM NaCl containing 1mM Phenylmethylsulphonyl fluoride (PMSF). Cell breakage was accomplished by a double passage through a pre-cooled French pressure cell (11.36 x 104kg (30,0001b)IU⁻). EDTA was added to broken cells to a final concentration of 5mM. Unbroken cells were removed by centrifugation at 17,000 x g for 15 mins. The extract was further clarified by acidification to pH 5.0 at room temperature.

The protein concentration was first adjusted to 10 mg/ml and then the extract titrated to pH 5.0 with 2M acetic acid. The sample was left stirring for 15 mins and then the precipitate removed by centrifugation at 17,000 x g for 15 mins. The supernatant was neutralised by addition of 1M Tris/HCl pH 8.0, dialysed into 25mM Tris/HCl pH 8.0, 10mM NaCl and applied to a DEAE-Sepharose (Trade Mark) column (2.6 x 8 cm). The column was eluted with a linear gradient of 10mM-400mH NaCl in 25mM Tris/HCl pH 8.0. Fractions containing fragment C were pooled and dialysed into 25mM Tris/HCl, 10mM NaCl. The dialysed sample was titrated to pH 5.2 with 2M acetic acid just prior to application to an S-Sepharose column (1.6 x 7cm) equilibrated with 25mM acetate pH 5.2. A fine precipitate was generated which was removed by centrifugation with little loss of fragment C.

After sample application the column was eluted with a linear gradient of 10mM-400mM NaCl. Fragment C containing fractions were pooled and dialysed into 25mM Tris/HCl pH 8.0, 10mM NaCl. The final chromatography was on a Q-Sepharose column (1.6 x 7 cm), elution again being achieved with a linear 10mM-400mM NaCl gradient in 25mM Tris/HCl pH 8.0. The peak fractions were pooled, concentrated and dialysed into 50mM Tris/HCl pH 7.6, 0.2M Nacl, 0.1mM EDTA samples at a final protein concentration of 2-3 mg/ml were stored either at 4°C or -70°C.

### Protein Assays

A two antibody sandwich enzyme-linked immunosorbent assay (ELISA) for fragment C was developed. Purified pepsinised antibody prepared from horses hyperimmunised with tetanus toxoid (Hughes et al J. Appl. Bact. 37, 1974, 603-622) was used to coat flexible polyvinylchloride microtitre plates (Dynatech, Billingshurst, GB). Coating was performed overnight at 4°C in 50 mM sodium carbonate pH 9.5 (12.5 µg pepsinised antibody per ml, 100µl per well). Plates were washed three times with phosphate buffered saline pH 7.2, 0.12 (w/v) Tween (Trade Mark) 20 (PBS-Tween). Non-specific binding was reduced by incubation of plates with PBS-Tween containing 12 (wt/vol) BSA or 5% (wt/vol) non-fat dry milk. Plates were then incubated sequentially with antigen, second antibody and anti-rat IgG peroxidase conjugate (Kinkegaard and Perry, Maryland, US) for 1 hr at 37°C in each case. The second antibody was a rat monoclonal (TT09) which binds with high affinity to fragment C (Sheppard et al, Infect. Immun. 43, 1984, 710-714) and it was used at a concentration of 2µg/ml.

Anti-rat peroxidase conjugate was used at a dilution of 1:3000. Each reagent was diluted into PBS-Tween containing 5% non-fat dry milk prior to addition to plates. Plates were washed three times with PBS-Tween after each incubation. Bound enzyme complex was assayed using tetramethyl benzidine (TMB) as the chromogenic substrate. lmg TMB tablets (Wellcome Diagnostics) were dissolved in 10ml of 0.0625M trisodium citrate containing 0.01% hydrogen peroxide. 100µl of reagent was added to each well and the reaction terminated by addition of 100µl 2M H₂SO₄ after incubation for 10-15 mins at room temperature. Plates were read using a Titertek multiskan plate reader (MCC/340) at 450nm.

For quantitation fragment C prepared from C.tetani (Fairweather et al, 1986) was used as a standard. Several dilutions of this fragment C gave a narrow range titration curve with linear portion between about 1µg/ml and 10µg/ml. Titration curves for the recombinant fragment C were comparable with similar slope and range to the standard curve. The titre of unknown samples was determined either by comparing the midpoints of titration curves or more routinely by reading directly from the linear portion of the standard curve. During purification, chromatographic fractions were screened for fragment C by immunodiffusion in 1% (wt/vol) agarose gels containing pepsinised horse antibody (approximately 0.25mg/ml). Protein was determined using the BCA assay reagent (Pierce) with bovine serum albumin as protein standard.

### 2. RESULTS

### Expression of fragment C in E.coli

Fragment C was expressed in E.coli using the two plasmids pTETtac1 and pTETtac2. pTETtacl encodes tetanus toxin residues 537 to 864 (a portion of fragment B) and residues 865 to 1315 (fragment C). The numbering of the residues is according to Eisel et al, EMBO J. 5, 2495-2502, 1986 and Fairweather and Lyness, Nuc. Acid Res. 14, 7809-7812, 1986. E.coli cultures harbouring pTETtac1 were induced for expression of the tetanus fragments and were analysed by sodium dodecyl sulphate-polyacrylamide gel electrophoresis (SDS-PAGE). pTETtac1 expressed moderate levels of a 90kD protein (Fig 2) as predicted from the nucleotide sequence of tetanus toxin (Fairweather and Lyness, 1986). Confirmation of this 90kD band as a tetanus toxin fragment was obtained by Western blotting against anti-fragment C sera (Fig 2). After breakage of the cells and high speed centrifugation of the extract, most of the 90 kD protein was found to be in the pellet fraction (Fig 2) as has been described for another fragment of tetanus toxin expressed in E.coli (Fairweather et al, 1986).

Plasmid pTETtac2 was predicted to express a 50kD polypeptide which exactly corresponds to fragment C but with an additional methionine residue at the N-terminus. As shown in Fig 2, induction of cells containing pTETtac2 resulted in expression of a protein of the correct size and which is recognised by anti-fragment C antisera (Fig 2). The level of expression of pTETtac2 was higher than that of pTETtac1. Essentially all of the product was found in the soluble fraction after high speed centrifugation of cell extracts (Fig 2). The levels of fragment C expressed by E.coli harbouring pTETtac2 and grown on a laboratory scale were determined by ELISA. Approximately 12 mg fragment C was obtained per litre of culture corresponding to approximately 3% total cell protein (Table 2).

### Purification and characterisation of fragment C produced in E.coli

Fragment C produced in E.coli was purified using a combination of acid precipitation and ion exchange steps. No evidence of any heterogeneity was observed during chromatography. The results of the purification procedure are summarized in Table 2. There were still some minor E.coli contaminants after the final step and the overall purity was estimated to be 85% by densitometry of SDS- polyacrylamide gel electrophoresis (Figure 3A, lane 2). The overall yield was about 50%.

Fragment C derived from E.coli and C.tetani were compared by electrophoretic analysis. Under non-reducing conditions on SDS-PAGE two bands of fragment C are seen in both preparations (Figure 3A, lanes 4 and 5). The less intensely staining band co-migrates with reduced fragment C (Figure 3A, lanes 2 and 3).

Preparations of recombinant fragment C showed a major Coomassie staining band on non-denaturing PAGE (Figure 3B, lane 1) and isoelectric focussing gels (data not shown). The pI was determined to be 7.2. One or two additional minor species having more acidic pI's were occasionally seen. Heterogeneity was more apparent in preparations stored for prolonged periods (4 weeks or more) at 4°C. Fragment C from C.tetani shows three Coomassie staining bands of similar intensity on non-denaturing electrophoresis (Figure 3B, lane 2) which co-migrate with the species seen in the recombinant preparation (lane 1). Such heterogeneity has previously been observed in fragment C from C.tetani (Helting and Zwisler, 1977) and was thought to be due to deamidation.

The immunogenicity of fragment C produced from E.coli and C.tetani was compared by immunising mice with vaccines containing fragment C followed by challenge with tetanus toxin. Table 1 shows that both preparations gave excellent protection against challenge, indicating similar protective potency of the two samples.

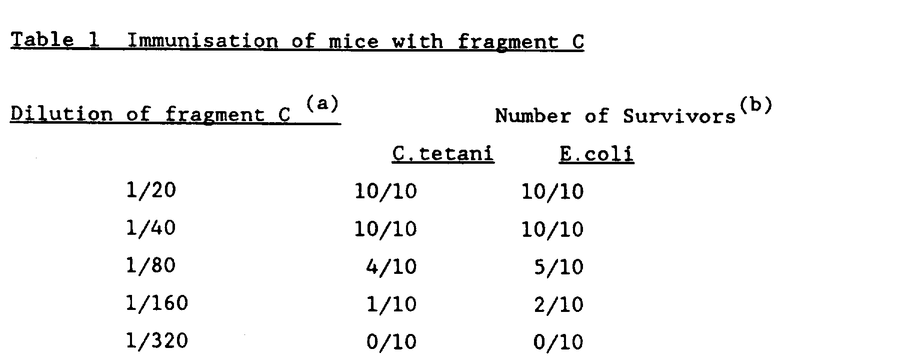

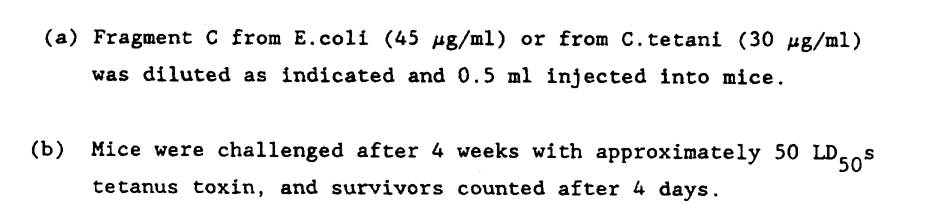

**Table 2**

| Purification of recombinant Fragment C | | | |
|---|---|---|---|
| Step | Total Protein (mg) | Fragment C (mg) | Recovery % |
| Total lysate | 1030.0 | 29.6 | 100 |
| Acid clarified extract | 622.0 | 25.5 | 86 |
| DEAE-Sepharose | 134.0 | 23.6 | 80 |
| S-Sepharose | 29.7 | 17.6 | 59 |
| Q-Sepharose | 15.5 | 15.7 | 53 |

### COMPARATIVE EXAMPLE

Expression plasmid pTet18 expresses a polypeptide which comprises Met-Asn-Ser-Arg-Gly followed by 121 residues of B fragment and all 451 carboxy-terminal residues of C fragment, i.e. residues Ser-744 to Asp-1315 of tetanus toxin (Fairweather et al, 1987). The solubility of this polypeptide in the cytoplasm of E.coli was assessed.

E.coli DHI carrying plasmid pTetl8 was grown overnight in L-broth containing ampicillin (100µg/ml). The cells were diluted 1 in 5 into fresh medium containing 60µg/ml IPTG (isopropyl-p-D-thiogalactopyranoside) and were grown for 4 hours. The cells from 2 litres of culture were harvested by centrifugation at 6,000 rpm for 20 mins with a Sorval GS3 rotor, and the cell pellets were stored at -20°c overnight. The pellets were thawed and suspended in a total of 32 ml.

## Claims

1. A process for producing fragment C of tetanus toxin in soluble form, which process comprises:
(i) culturing an Escherichia coli host cell transformed with an expression vector comprising a coding sequence consisting of a Met start codon followed by a sequence encoding fragment C, wherein said culturing is effected under conditions such that said coding sequence is expressed and fragment C thereby produced in soluble form; and
(ii) recovering said soluble fragment C from the cytoplasm of said Escherichia coli host cell.

2. A process according to claim 1 wherein the amino acid sequence of said soluble fragment C has more than 90% homology with that set forth in Figure 4.

3. A process according to claim 1 wherein the amino acid sequence of said soluble fragment C has more than 95% homology with that set forth in Figure 4.

4. A process according to claim 1 wherein the amino acid sequence of said soluble fragment C has more than 98% homology with that set forth in Figure 4.

5. A process according to claim 1 wherein said soluble fragment C has the amino acid sequence set forth in Figure 4.

6. A process according to any one of the preceding claims wherein said Escherichia coli host cell is selected from strains HB101, DH1 and MM294.

7. An Escherichia coli expression vector comprising a coding sequence consisting of a Met start codon followed by a sequence encoding fragment C of tetanus toxin.

8. A vector according to claim 7 wherein said fragment C has an amino acid sequence as defined in any one of claims 2 to 5.

9. An Escherichia coli host cell transformed with an expression vector as claimed in claim 7 or 8.

10. An Escherichia coli host cell according to claim 9 selected from strains HB101, DH1 and MM294.

## Patentansprüche

1. Verfahren zur Herstellung des Fragmentes C vom Tetanustoxin in löslicher Form, wobei das Verfahren folgendes umfaßt:
(i) Kultivieren einer Escherichia coli-Wirtszelle, transformiert mit einem Expressionsvektor, der eine kodierende Sequenz umfaßt, bestehend aus einem Met-Startkodon, gefolgt von einer Sequenz, die für das Fragment C kodiert, wobei die Kultivierung unter solchen Bedingungen ausgeführt wird, daß die kodierende Sequenz exprimiert wird und das Fragment C dadurch in löslicher Form gebildet wird; und
(ii) Gewinnen des löslichen Fragmentes C aus dem Zytoplasma der Escherichia coli-Wirtszelle.

2. Verfahren nach Anspruch 1, wobei die Aminosäuresequenz des löslichen Fragmentes C mehr als 90% Homologie mit der in Figur 4 dargelegten besitzt.

3. Verfahren nach Anspruch 1, wobei die Aminosäuresequenz des löslichen Fragmentes C mehr als 95% Homologie mit der in Figur 4 dargelegten besitzt.

4. Verfahren nach Anspruch 1, wobei die Aminosäuresequenz des löslichen Fragmentes C mehr als 98% Homologie mit der in Figur 4 dargelegten besitzt.

5. Verfahren nach Anspruch 1, wobei das besagte lösliche Fragment C die Aminosäuresequenz, die in Figur 4 dargelegt wird, besitzt.

6. Verfahren nach einem der vorhergehenden Ansprüche, wobei die Escherichia coli-Wirtszelle aus den Stämmen HB101, DH1 und MM294 gewählt wird.

7. Escherichia coli-Expressionsvektor, der eine kodierende Sequenz, bestehend aus einem Met-Startkodon, gefolgt von einer Sequenz, die für das Fragment C vom Tetanustoxin kodiert, umfaßt

8. Vektor nach Anspruch 7, wobei das Fragment C eine Aminosäuresequenz besitzt, wie sie in einem der Ansprüche 2 bis 5 definiert ist.

9. Escherichia coli-Wirtszefle, transformiert mit einem Expressionsvektor, wie er in Anspruch 7 oder 8 beansprucht wird.

10. Escherichia coli-Wirtszelle nach Anspruch 9, ausgewählt aus den Stämmen HB101, DH1 und MM294.

## Revendications

1. Procédé de production du fragment C de la toxine tétanique sous une forme soluble, procédé qui comprend les étapes consistant à :
(i) cultiver une cellule hôte d'Escherichia coli transformée avec un vecteur d'expression comprenant une séquence codante consistant en un codon d'initiation Met suivi d'une séquence codant pour le fragment C, où ladite culture est effectuée dans des conditions telles que ladite séquence codante soit exprimée et le fragment C soit ainsi produit sous une forme soluble, et
(ii) récupérer ledit fragment C soluble à partir du cytoplasme de ladite cellule hôte d' Escherichia coli.

2. Procédé selon la revendication 1 dans lequel la séquence d'acides aminés dudit fragment C soluble a une homologie de plus de 90 % avec celle présentée à la Figure 4.

3. Procédé selon la revendication 1 dans lequel la séquence d'acides aminés dudit fragment C soluble a une homologie de plus de 95 % avec celle présentée à la Figure 4.

4. Procédé selon la revendication 1 dans lequel la séquence d'acides aminés dudit fragment C soluble a une homologie de plus de 98 % avec celle présentée à la Figure 4.

5. Procédé selon la revendication dans lequel ledit fragment C soluble a la séquence d'acides aminés présentée à la Figure 4.

6. Procédé selon l'une quelconque des revendications précédentes dans lequel ladite cellule hôte d'Escherichia coli est choisie parmi les souches HB10l, DH1 et MM294.

7. Vecteur d'expression dans Escherichia coli comprenant une séquence codante consistant en un codon d'initiation Met suivi d'une séquence codant pour le fragment C de la toxine tétanique.

8. Vecteur selon la revendication 7 dans lequel ledit fragment C a une séquence d'acides aminés telle que définie dans l'une quelconque des revendications 2 à 5.

9. Cellule hôte d' Escherichia coli transformée avec un vecteur d'expression tel que revendiqué dans la revendication 7 ou 8.

10. Cellule hôte d' Escherichia coli selon la revendication 9 choisie parmi les souches HB101, DH1 et MM294.
